**Europäisches Patentamt**

**European Patent Office**

**Office européen des brevets**

(19)

(11) Veröffentlichungsnummer: **0 120 117**
**B1**

## EUROPÄISCHE PATENTSCHRIFT

(12)

(45) Veröffentlichungstag der Patentschrift:
13.08.86

(21) Anmeldenummer: 83108286.2

(22) Anmeldetag: 22.08.83

(51) Int. Cl.⁴: **A 61 F 13/00**, D 04 H 5/06,
**B 32 B 7/00**

(54) Elastische Bandage zur Fixierung von Körperteilen und Verfahren zu ihrer Herstellung.

(30) Priorität: 23.03.83 DE 3310527

(43) Veröffentlichungstag der Anmeldung:
03.10.84 Patentblatt 84/40

(45) Bekanntmachung des Hinweises auf die Patenterteilung:
13.08.86 Patentblatt 86/33

(84) Benannte Vertragsstaaten:
BE DE FR GB IT NL SE

(56) Entgegenhaltungen:
EP-A-0 045 592
DE-B-1 491 205
DE-B-1 760 436
US-A-2 740 402

(73) Patentinhaber: Firma Carl Freudenberg, Höhnerweg 4, D-6940 Weinheim/Bergstrasse (DE)

(72) Erfinder: Tecl, Bohuslav, Im Langgewann 57,
D-6940 Weinheim (DE)
Erfinder: Groitzsch, Dieter, Dr., Hermann- Löns-
Strasse 6A, D-6945 Hirschberg 2 (DE)
Erfinder: Fahrbach, Erich, Dr., Zinkgräfstrasse 62,
D-6940 Weinheim (DE)

(74) Vertreter: Weissenfeld- Richters, Helga, Dr.,
Höhnerweg 2, D-6940 Weinheim/Bergstrasse (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine elastische Bandage zur Fixierung von Körperteilen, bestehend in aus Zugrichtung der Bandage parallel zueinander angeordneten und wenigstens einseitig durch textile Flächengebilde abgedeckte gedehnte Elastomerfäden, wobei die Elastomerfäden mit dem bzw. den textilen Flächengebilde(n) bzw. die textilen Flächengebilde miteinander verbunden bzw. verklebt sind und die Bandage infolge Relaxation der eingebundenen Elastomerfäden gestaucht ist. Die Erfindung betrifft weiterhin ein Verfahren zur Herstellung derartiger Bandagen.

Elastische Bandagen sind bekannt. Sie sind in der Regel durch Vereinigung wenigstens einer meist textilen Stoffbahn mit gedehnten hochelastischen Fäden und Relaxation der Fäden unter Wellung der Stoffbahn hergestellt. Die Dehnbarkeit kann durch entsprechende Auswahl der elastischen Elemente variiert werden. So unterscheidet man Kurz-Mittel- und Langzugbandagen. Insbesondere bei langer und mittlerer Dehnbarkeit besteht die Gefahr, daß das Verbandmaterial einschnürt, d.h. bei Anwendung verringert sich die Breite der Bandage. Diese Gefahr besteht insbesondere bei Bandagen auf Vliesstoff- und Folienbasis und es sind zahlreiche Vorschläge gemacht worden zur Entwicklung von Bandagen, die einerseits unkompliziert in der Handhabung und hautfreundlich sind und andererseits die erforderliche Dehnung und Festigkeit bei möglichst geringer Einschnürung des behandelten Körperteils gewährleisten.

DE-N-77 25 500 beschreibt eine elastische Bandage mit kurzer bis mittelmäßiger Dehnbarkeit, die aus einem hochelastischen Gewebe besteht, dessen Kettfäden umsponnene Polyurethan- oder Gummifäden sind, während die Schußfäden zur Erhöhung der Hautfreundlichkeit und des Tragekomforts aus Baumwolle, Zellwolle oder sonstigen hydrophilen Naturfasern bestehen. Das Dehnungsverhalten ist durch die elastischen Kettfäden bestimmt. Die Unabhängigkeit von Kette und Schuß zueinander innerhalb des Gewebes hat zur Folge, daß bei Dehnung der Bandage kein Breitenschwund und somit keine Einschnürung des behandelten Körperteiles eintritt. Die Elastomerfäden müssen wegen der geforderten Festigkeit relativ dick sein. Entsprechendes gilt für die Schußfäden, so daß die Bandage nicht den erforderlichen Tragekomfort aufweist. Es ist weiterhin nachteilig, daß beim Zuschneiden des Materials die Schnittränder ausfransen und entweder vernäht oder verklebt werden müssen, wodurch die Dehnbarkeit negativ beeinflußt wird und überdies mit Druckstellen gerechnet werden muß.

Zur Verbesserung der Trageeigenschaften ist deshalb vorgeschlagen worden, die gedehnten Elastomerfäden nicht in ein Gewebe einzuarbeiten, sondern in Zugrichtung parallel nebeneinander angeordnet zwischen Stoffbahnen zu fixieren. DE-B-17 60 436 beschreibt ein derartiges elastisches Bandagematerial, das aus gedehnten hochelastischen Fäden besteht, die mit wenigstens einer textilen Stoffbahn durch Verkleben mit Maturkautschuk-Latex enthaltenden polymeren Bindemitteln vereinigt sind. Zur Vermeidung der bei Geweben bekannten Nachteile wird hier vorgeschlagen, daß wenigstens eine der textilen Stoffbahnen aus einem imprägnierten Wirrfaservliesstoff besteht. Die Fäden werden in gedehntem Zustand mit der Stoffbahn vollflächig verklebt und anschließend durch Relaxation verkürzt, wobei eine Wellung des Materials eintritt. Dies ist notwendig, weil bei der Verklebung ungedehnter elastischer Fäden mit der Stoffbahn, z.B. dem Vliesstoff, keine elastisch verformbaren Bandagen ergeben würde. Die Stoffbahn bzw. der Vliesstoff sind weitgehend unelastisch und würden die Dehnung der Elastomerfäden verhindern. Es ist somit notwendig, die Elastomerfäden stark, d.h. um mehr als 100 % zu dehnen und in diesem Zustand zwischen die bereits verfestigten, latexgebundenen Vliesstoffe abzulegen. In diesem Zustand werden die Elastomerfäden vollflächig mit dem bereits vorher latexgebundenen Vliesstoff verklebt und nach dem Trocknen entspannt. Es entsteht so ein gewelltes, elastisches Bandagenmaterial. Infolge der starken Dehnung und der Entspannung des vollflächig verklebten relativ steifen Materials entsteht eine papierartige Bahn mit wenig textilem Charakter. Man ist aus diesem Grunde wieder davon abgegangen, Vliesstoffe zu elastischen Bandagen zu verarbeiten.

Der Ersatz von Gewebestoffbahnen durch Folien,wie er z.B. in EP-A-0 045 592 beschrieben ist, hat ein elastisches Bandagenmaterial ergeben, das aus einer Schicht aus einem Elastomerfilm besteht mit ein oder zwei Außenschichten aus Vliesstoff oder anderen dehnbaren Flächengebilden, wie Netzen oder dgl. Der Elastomerfilm wird ein- oder zweiseitig mit Haftmasse bedruckt, stark gedehnt und im gedehnten Zustand ein- bzw. beidseitig auf die textilen Flächengebilde kaschiert und dann wieder entlastet. Es ist offensichtlich, daß Folien, auch wenn sie geschlitzt oder gelocht sind, beim Dehnen einschnüren und dies um so stärker je höher die Dehnung ist. Aus diesem Grunde sind Bandagen dieser Art insbesondere als therapierende mittel- oder langzügige Kompressionsbandage nicht brauchbar. Es ist weiterhin nachteilig, daß derartige Laminate nicht waschbeständig sind.

Neben der Nichtwaschbarkeit und der Gefahr des Einschnürens einer Bandage nach EP-A-0 045 592 macht sich die mangelnde Luft- und Feuchtigkeitsdurchlässigkeit nachteilig bemerkbar. Dies führt zu Hitzestau und Mazerationseffekten selbst, wenn die elastischen Folien mit Öffnungen versehen sind. Es besteht weiterhin die Gefahr, daß beim Wickeln der Bandage die Öffnungen der Folie durch geschlossene Folienbereiche der überlappenden Lagen völlig abgedichtet werden. Der damit nahezu vollständig unterbundene Luftbzw.

Feuchtigkeitsaustausch wirkt begünstigend auf das Wachstum von Mikroorganismen wie Bakterien, Sporen, Pilze oder dgl.

Der Erfindung liegt nun die Aufgabe zugrunde, eine luft- und feuchtigkeitsdurchlässige waschbare Bandage zu entwickeln, die nicht einschnürt. Es ist weiterhin erwünscht, daß die Schnittränder nicht besonders geschützt bzw. bearbeitet werden müssen. Die Bandage soll somit im wesentlichen die an sich bekannten günstigen Eigenschaften der Gewebebandagen aufweisen, insbesondere deren hohen Kompressionsdruck. Es sollen jedoch die Nachteile der Gewebebandagen vermieden werden, insbesondere deren relativ rauhe Oberfläche, die durch den bei der Herstellung straffer Mittelzug- oder Langzugbandagen notwendigen Elastomerfadentiter über 1000 dtex oder denier verursacht ist. Da derart dicke Kettfäden durch die Schußfäden nur teilweise abgedeckt werden, sind Abdrücke und Reizungen der Haut unvermeidlich.

Die erfindungsgemäße Aufgabe wird durch die in den Patentansprüchen definierte elastische Bandage gelöst. Die weiterhin gestellte Aufgabe nach einem besonders günstigen Herstellungsverfahren wird ebenfalls durch das in den entsprechenden Patentansprüchen wiedergegebene Verfahren gelöst.

Die gedehnten und ein- oder mehrfach umsponnenen Elastomerfäden sind in Zugrichtung parallel zueinander angeordnet und ein- oder beidseitig mit einem möglichst leichtbereist leicht vorgebunden sein. Ein besonders vorteilhaftes Verfahren zur Herstellung der Bandage besteht jedoch darin daß die umsponnenen gedehnten Elastomerfäden zwischen nicht gebundenen Faserfloren geführt und dann gleichzeitig mit der stellenweisen thermoplastischen Verschweißung der Flore zu einem Mehrschichtgebilde auf Vliesstoffbasis fest eingebunden werden. Die Elastomerfäden sind in ihrer Lage fixiert und trotzdem gut beweglich, weil sie sich über mehr oder weniger große Bereiche an den nicht gebundenen Stellen des Vliesstoffes befinden. Auf diese Weise wird eine optimale Beweglichkeit und Dehnbarkeit des gesamten Bandagenmaterials und zwar sowohl der Abdeckvliesstoffe als auch der Elastomerfäden erreicht. Die Schnittstellen sind nahezu unproblematisch, weil ein Ausfransen wegen der stellenweisen Verfestigung entfällt.

Während die Höhe der elastischen Zugkraft bei Gewebebandagen und gegebener Gewebeart vom Titer der Elastomermonofilfäden bestimmt wird - mit steigendem Titer zunehmend Zugkraft - sind Gewebebandagen mit relativ hohem Kompressionsdruck besonders rauh und unangenehm. Elastische Bandagen auf Vliesstoffbasis gemäß der vorliegenden Erfindung bestehen dagegen aus einwandfrei mit einem weichen, elastischen, luftdurchlässigen und leichten Material abgedeckten Elastomerfäden, die unabhängig von der Zugkraft stets eine formbeständige und druckfrei komprimierende Bandage gewährleisten. Die zweckmäßig beidseitig mit dem Vliesstoff abgedeckten umsponnenen Elastomerfäden ergeben einen Kompositvliesstoff. Der Vliesstoff kann vor dem Kaschieren bereits in gebundenem Zustand vorliegen, jedoch ist es für die Herstellung besonders hautangenehmer Bandagen zweckmäßig, nicht verfestigte lose Faserflore zu einem Flächengebilde abzulegen und die umsponnenen Elastomerfäden damit abzudecken. Die Verbindung der Elastomerfäden mit den Faserfloren zu dem Kompositvliesstoff erfolgt durch die erwähnte, nicht vollflächige thermische Verschweißung z.B. mit Hilfe eines Kalanders der eine beheizte Prägewalze mit dem erwünschten Rastermuster enthält.

Die Vliesstoffe bzw. Faserflore enthalten zweckmäßig wenigstens 10 Gew.%, bezogen auf das Gesamtgewicht der Fasern, an thermoplastischen Bindefasern. Bindefasern dieser Art sind an sich bekannt. Gewöhnliche schmelzgesponnene Homofilfasern bestehen beispielsweise aus Polyamid 6, Polyamid 66, Copolyamiden, Polyestern, Polypropylen und dessen Copolymeren. Für besonders weiche Produkte ist es vorteilhaft, Mehrkomponenten-Heterofilfasern zu verwenden. Es eignen sich beispielsweise Bikomponenten-Fasern aus Polypropylen und Polyäthylenvinylacetat, Polypropylen und Polyäthylen, Polyester und Polyäthylen, Polyamid 66 und Polyamid 6 oder Polyester und Copolyester.

Je nach den gewünschten Eigenschaften des fertigen Bandagenmaterials wird der Anteil an thermoplastischen Fasern zwischen 10 und 100 Gew.% betragen. In machen Fällen ist es vorteilhaft, wenn die die Elastomerfäden abdeckenden Faservliese über die Flordicke hindurch nicht homogen aufgebaut sind. So ist es zweckmäßig, auf der Außenseite der Bandage bis zu 100 Gew.% Bindefasern zu verwenden. Dies läßt sich verfahrensmäßig dadurch bewerkstelligen, daß zur Vlieslegung mehrere Krempeln und/oder Karden eingesetzt werden. Die einzelnen Krempeln oder Karden werden dann mit unterschiedlichen Fasermischungen beschickt. Ein zwei- oder mehrschichtiger Vliesstoffaufbau ist auch dann zweckmäßig, wenn ein hoher Oberflächenabrieb und eine gute Waschbarkeit mit großer Weichheit verbunden werden soll.

In diesem Falle enthält die Außenseite einen hohen Bindefaseranteil, während die den Elastomerfäden zugewandte Innenseite einen niedrigen Bindefaseranteil enthält und somit die Weichheit der Bandage gewährleistet.

Die Abdeckvliesstoffe bzw. -flore werden wirr-, quer- oder längsgelegt und werden in Abhängigkeit von dem späteren Verwendungszweck nach unterschiedlichsten Vliesstofftechnologien hergestellt. So ist es vielfach zweckmäßig Spinnvliesstoffe, Naßvliesstoffe und/oder Trockenvliesstoffe in entsprechender Kombination zu vereininen. Zweckmäßig ist z.B. die gemeinsame Verwendung quergelegter Flore mit einem oder mehreren Längsfloren. Derartige Vliesstoffe werden auch "criss/cross-gelegte" Vliesstoffe genannt. Längsflore als Außenlage sind immer dann vorteilhaft, wenn eine besonders glatte Oberfläche gewünscht ist.

Das Bandagenmaterial läßt sich beliebig, z.B. hautfarbig einfärben. Es ist weiterhin möglich, sonstige Zusätze, z.B. antibakterielle, antimykotische oder sonstige Zusätze vorzunehmen.

Der nicht umsponnene Elastomerfaden liegt in einem Titerbereich von 20 bis 2200 denier. Durch ein- oder zweimalige Umspinnung und gegebenenfalls Variation der Umspinnbedingungen wird die elastische Dehnung beliebig, zweckmäßig auf Beträge zwischen 30 und 400 % eingestellt. Der Kern des umsponnenen Elastomerfadens besteht zweckmäßig aus Gummi oder Polyurethan, z.B. einem unter der Bezeichnung "Lycra-Spandex" von Du Pont vertriebenen Material. Der Elastomermonofilfaden wird mit zwei Polyfil-Zwirnfäden mit Z- und S-Drehungsrichtung oder nur einseitig parallel nach dem sogenannten "Coretwist"-Verfahren umzwirnt sein. In beiden Fällen kann die Umspinnung aus einem Einfach- oder Doppelgarn bestehen. Dieses kann wiederum glatt oder texturiert sein.

Das Fasermaterial der Zwirnfäden besteht aus Nylon, Perlon, Polyester, Zellwolle, Baumwolle oder Mischungen aus Baumwolle und Polyester bzw. Zellwolle und Polyester. Die Umspinnung kann mit jedem gewünschten Fasermaterial erfolgen. Neben Homofilfasern aus Homo- oder Copolymerisaten bzw. -Polycondensaten sind auch Umspinnungsgarnen aus Heterofilfasern, in der Regel Bikomponentenfasern, möglich. Die Zwirnfäden bestehen zweckmäßig aus thermoplastischen, der Hitzeverschweißung zugänglichen, zusammen mit unschmelzbaren Fasern.

Für die Herstellung der Bandagen ist es zum Zwecke der besseren Einbindung der Elastomerfäden in die sie abdeckenden Vliesstoffschichten vorteilhaft, wenn die Zwirnfäden aus thermoplastischen Fasern mit nicht zu hohem Schmelz- bzw. Erweichungspunkt bestehen. Aus diesem Grunde sind Perlon- und Bikomponentenfasern mit Perlon als Schmelzklebekomponente sowie Perlon oder Copolymere mit einem Schmelz- bzw. Erweichungsbereich unter dem des Perlons und entsprechende Bikomponentenfasern bevorzugt.

Der Titer des nicht umsponnenen Elastomerfadens liegt im Bereich von 20 bis 2200 denier. Je nach Struktur der Umspinnung und des erwünschten elastischen Dehnungsbetrages kann der Anteil des reinen Elastomerfadens am Gesamtgewicht des umsponnenen Fadens variiert werden. Gummifäden haben bei gleichem Titer eine geringere Kompressionskraft als Polyurethanfäden. Zur Erzielung einer bestimmten Kompressionskraft ist somit bei Polyurethan ein niedrigerer Titer und damit ein niedrigeres Gewicht notwendig als bei Gummifäden. Polyurethan wird überdies wegen der besseren Wasch-, Alterungs-, Schweiß- und Chlorbeständigkeit gegenüber Gummi bevorzugt.

Der Abstand der Elastomerfäden variiert zwischen 1 und 15 mm. Zweckmäßig wird ein Abstand von 2 bis 10 mm eingehalten.

Die umsponnenen Elastomerfäden weisen eine Dehnung von 30 bis 400 % auf. Die elastische Dehnung wird durch die Vorspannung des Elastomerfadens, den Umspinnungsprozeß und die Geometrie der Umspinnung eingestellt.

Die Dehnung der fertigen Bandage und die Kompressionskraft werden beeinflußt durch den Elastomerfadentiter, den Dehnungsbetrag der eingelagerten Elastomerfäden, die Faserflorgewichte und die Faserzusammensetzung der Abdeckvliese bzw. Vliesstoffe, das Rastermuster und die Gravurtiefe und die Form der verschweißten Stellen sowie den prozentualen Anteil der Verschweißfläche an der Gesamtfläche. Hierdurch lassen sich langzügige, mittelzügige und kurzzügige Kompressionsbandagen mit Dehnungen von 140 bis 200, 70 bis 140 bzw. 30 bis 70 % bei 1 cm Streifenbreite und 1 kg Belastung nach DIN 61 632 herstellen.

Die Bandage findet vorzugsweise Anwendung als Kompressionsbandage nach Distorsionen, Frakturen, Luxationen und vorbeugend gegen Sportverletzungen, jedoch auch als Fixierbinde und als Schutzverband. Weitere Anwendungen ergeben sich bei der Varizenverödung, chronischen und entzündlichen Venenstauungen, bei lymphardischen Ödemen, Ulcara cruris, Stauungsdermatosen und allen Erscheinungen des varikösen Symptomenkomplexes.

Nach der Einbindung des Elastomerfadens in den Vliesstoff wird der ursprüngliche, durch die Umzwirnung festgegelegte Dehnungsbetrag nicht mehr erreicht. Je nach qualitativer und quantitativer Zusammensetzung des Kompositvliesstoffes und der Verfestigungsbedingungen wird ein Dehungsverlust erreicht. Diese Einbuße an Dehnung wird bei der Herstellung in Abhängigkeit vom Anwendungsfall einkalkuliert.

Die Bandage läßt sich ohne Gefahr des Ausfransens schneiden. Da jedoch an den Schnittflächen möglicherweise die Gefahr einer Löslösung der Elastomerfäden besteht, ist es vielfach zweckmäßig, die elastische Bandage an den Enden unmittelbar vor oder nach dem Querschneiden im Zusammenhang mit der Konfektionierung stegartig zu verschweißen. Die Verschweißung wird thermisch durch Ultraschall mit Hochfrequenz oder anderen Energiequellen bzw. Energieformen vorgenommen.

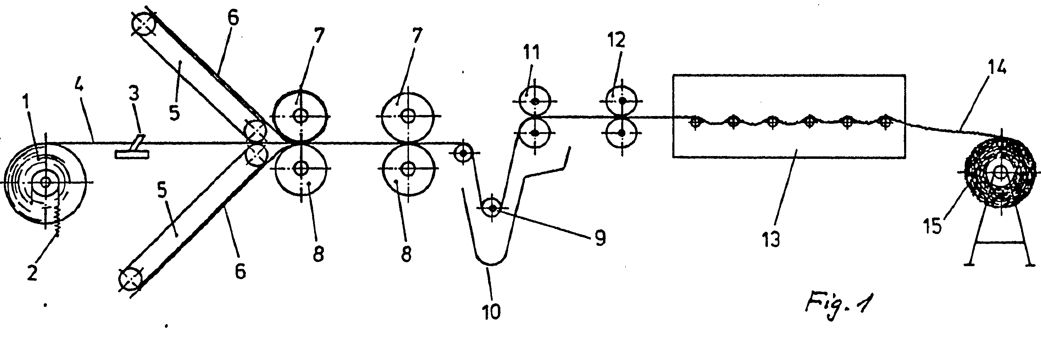

Figur 1 bis 3 zeigt die Herstellung der Bandagen bzw. deren Struktur.

Figur 1 ist ein Herstellungsschema für das elastische Bahnenmaterial. Die voll ausgedehnten, auf einem Kettbaum 1 aufgewickelten, umsponnenen Elastomerfäden werden unter Zugspannung, die durch eine Bremse 2 erzielt wird, über einen Kamm 3 abgewickelt. Über die Lattenbänder 5 einer Vlieslegemaschine werden die umsponnenen Gummi- oder Polyurethanfäden mit jeweils einem losen Faserflor 6 beidseitig abgedeckt. Die Verfestigung erfolgt mit Hitze und Druck in einem Kalander, der eine gravierte, beheizte Kalanderwalze 7 und eine glatte, beheizte Stahlwalze 8 aufweist. Falls die Elastomerfäden mit bereits vorgebundenen Vliesstoffen abgedeckt werden sollen, so erfolgt dies in der gleichen Verfahrensstufe, bei der die losen Faserflore 6 mit Hilfe der Lattenbänder 5 der Vlieslegemaschine abgelegt werden.

Nach der Hitzekalandrierung liegt nur ein Großteil der insgesamt erreichbaren elastischen Dehnung vor. Die verbleibende Restdehnung des Bandagenmaterials ist durch die Hitzekalandrierung quasi eingefroren bzw. fixiert worden und wird gegebenenfalls durch den anschließenden Prozeß ausgelöst werden. Die gesamte elastische Dehnung wird anschließend dadurch erreicht, daß das entlastete ungespannte Bahnenmaterial eine wäßrige Flotte 9 durchläuft. Hierzu wird es durch die gefüllte Tauchwanne 10 den Quetschwalzen II des Foulards zugeführt. Die wässrige Flotte besteht im einfachsten Fall nur aus Wasser. Es ist jedoch zweckmäßig, der Flotte Antischaummittel, Tenside, Mikrobizide, bakteriostatisch wirksame Substanzen, Textilweichmacher, Griffvariationen, den Griff nicht verhärtende Textilhilfsmittel, wäßrige Kunststoff-Dispersionen oder dgl. zuzusetzen. Die Flotte enthält weiterhin gegebenenfalls auf das Fasergut aufziehende Farbstoffe.

Das Material kann gegebenenfalls nach dem Abquetschen überschüssiger Flotte vor dem Kurschleifentrockner 13 naß-in-naß auf einer Druckanlage 12 bedruckt werden. Dies ist für eine besonders gute Einbindung der Elastomerfäden von Vorteil. Zur Bedruckung werden beispielsweise Tiefdruck- oder Siebschablonendruck-Verfahren angewendet.

Nach dem Abquetschen und gegebenenfalls Bedrucken passiert das feuchte Textilgut den Kurzschleifentrockner 13, der eine spannungsfreie Schrumpfung des Materials erlaubt. Durch die feuchtthermische Behandlung schrumpft und trocknet das Material. Das Endmaterial 14 erhält dadurch die erwünschten elastischen Eigenschaften. Es wird anschließend auf die Rolle 15 gewickelt.

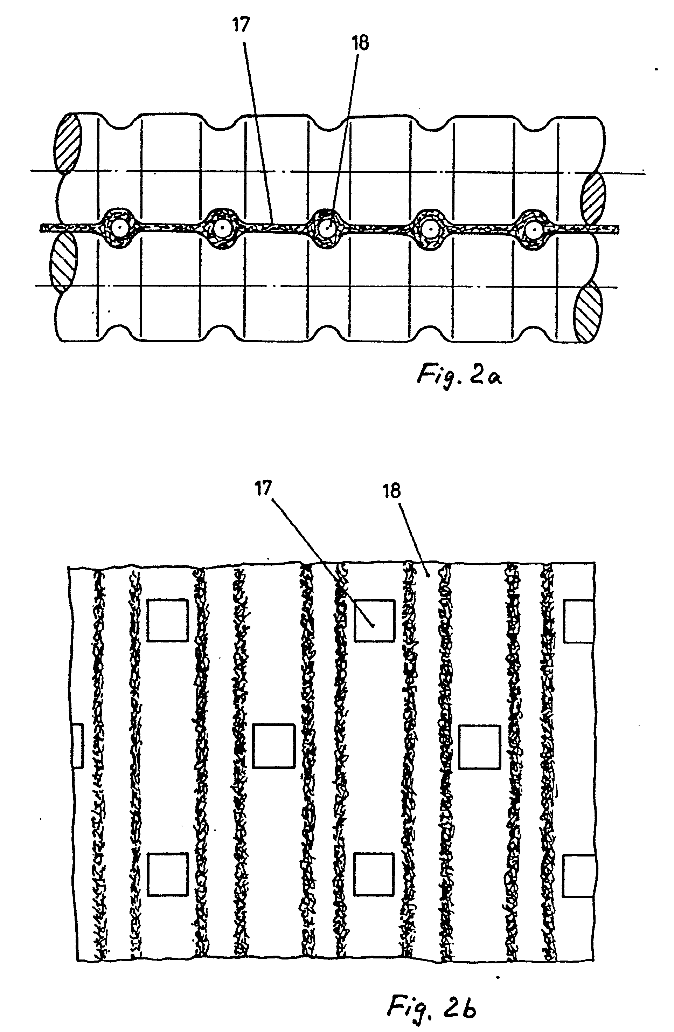

Figur 2a zeigt einen Querschnitt des Bandagenmaterials.

Figur 2b zeigt die Aufsicht des gleichen Bandagenmaterials.

In den Figuren 2a und 2b sind die infolge der Kalandrierung verdichteten und verschweißten Vliesstoffbereiche 17 sichtbar. In Abhängigkeit von dem Verdichtungs- und Verschweißungsgrad werden die Stellen 17 mehr oder weniger transparent. Der elastische Monofilfaden 18 ist zwischen den mehr oder weniger transparenten Verschweißfensterchen 17 eingebettet. Die Form der Verschweißfensterchen, deren Verteilung in der Fläche und der Anteil verschweißter Stellen in der gesamten Fläche ist variabel.

Die Verschweißflächen betragen in der Regel 4 bis 50 der Gesamtfläche, zweckmäßig jedoch zwischen 5 und 25 %. Bei der Wahl geeigneter Werte ist zu berücksichtigen, daß die Festigkeit durch zu niedrige Verschweißflächen beeinträchtigt wird und zu hohe Verschweißanteile einen gegebenenfalls ungünstig harten Griff ergeben.

Durch die Verschweißfensterchen 17 bzw. deren Verteilungsmuster werden die Elastomerfäden derart eingebunden, daß sie teilweise infolge der thermoplastischen Fasern der Umspinnung in die Verschweißung der Abdeckvliesstoffe einbezogen werden. Die Elastomerfäden werden somit fest verankert und bleiben trotzdem beweglich. Im Vergleich zu einer vollflächigen Verklebung ergibt sich eine optimale Festigkeit einerseits und Beweglichkeit andererseits der die Eigenschaften der Bandage beeinflussen den Elastomerfäden.

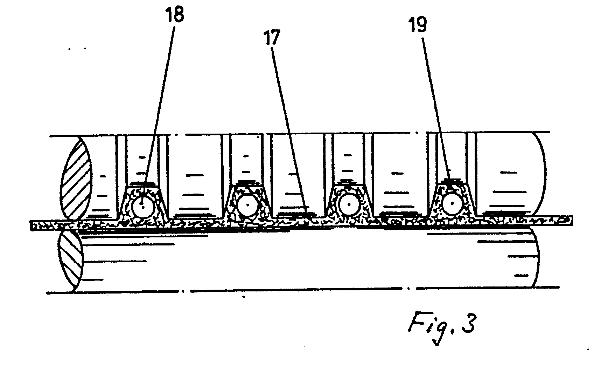

Figur 3 zeigt die Verschweißung des Bandagenmaterials (Ausschnitt) mit Hilfe einer Punktschweiß-Rasterwalze. Die umsponnenen Elastomerfäden 18 sowie eine der beiden Florlagen 19 werden durch die andrückende glatte (hier nicht eingezeichnete) Stahlwalze in die Zwischenräume der rechteckigen, pyramidenförmig erhöhten Stellen 17 gedrückt. An den Stellen 17 erfolgt die Verdichtung und Verschweißung des Fasermaterials zu den mehr oder weniger transparenten Fensterchen. Durch die Beheizung der Rasterwalze kommt es auch teilweise zum Anschmelzen oder Erweichung der Umzwirnung des Elastomerfadens, sofern dieser thermoplastisches Material enthält.

Die nachfolgenden Beispiele beschreiben die Herstellung des Bandagenmaterials.

**Beispiel 1**

Es wird eine elastische Bandage hergestellt, die auf 1 cm Breite 5 parallele, längsorientierte Lycra-Fäden, 940 dtex, mit Umspinnung zweimal dtex 100/26/2 und einer Dehnung von 170 % angeordnet. Die voll angespannte Längsfadenschar ist beidseitig mit je einer 30 g/m² schweren Vliesschicht aus folgenden Fasern abgedeckt:

15 % Bikomponentenfaser 3,3 dtex bestehend aus Polyamid 66-Kern und Polyamid 6-Mantel,

35 % Nylon 1,7 dtex

30 % Nylon 3,3 dtex

20 % Polyester 3,3 dtex.

Die beiden Vlieslagen werden in den Zwischenräumen der Längsfäden mit einer Teilung von 2 mm punktförmig verschweißt, wobei die einzelnen Schweißpunkte versetzt sind. Die Schweißpunkte sind in Längsrichtung 0,55 mm und in Querrichtung 0,80 mm lang. Bei einer Punktteilung in Längsrichtung von 1,43 mm sind die Schweißpunkte versetzt.

Die verschweißten Flächen haben, bezogen auf das Gesamtgewicht der elastischen Bandage, einen Anteil von 7 %. Jeder Faden ist auf beiden Seiten von Fasern, die in die Schweißstellen verankert sind, umhüllt. Durch das Zusammenwirken der Faserspannung und des Reibungskoeffizienten zwischen Faden und Faseroberfläche werden die Fäden in ihrer Lage fixiert. Die Schweißung erfolgt bei 228° C.

Zur Entspannung werden die Fäden durch Wasser bei 20° C gezogen.

Anschließend wird das Material in einem Kurzschleifentrockner bei 150° C getrocknet. Hierbei werden die Fäden entspannt und das gesamte Gebilde geschrumpft. Die elastische Bandage ist bis 70 % dehnbar. Sie ist als Kurz- und Mittelzugbandage im medizinischen Bereich verwendbar. Unter Dehung der Bandage wird die Längenveränderung ohne Einschnürung verstanden.

**Beispiel 2**

Es wird eine elastische Bandage gemäß Beispiel 1 hergestellt, wobei der Anteil an Bikomponentenfasern auf 45 erhöht wird. Die Bandage weist einen "kernigen" Griff und eine verbesserte Waschbeständiqkeit auf.

**Beispiel 3**

Nach Beispiel 1 wird eine elastische Bandage gefertigt, wobei die eingelegten, längsorientierten Fäden Polyurethanfäden von 156 dtex sind, die zweimal mit dtex 78/17/1 Nylon texturiert umsponnen sind. Es ergibt sich ein Endtiter von 205 dtex und eine Dehnung vom 40 %. Nach der Verarbeitung gemäß Beispiel 1 wird eine Kurzzugbandage erhalten, die zur Fixierung von Körperteilen verwendet wird.

**Patentansprüche**

1. Elastische Bandage zur Fixierung von Körperteilen, bestehend aus in Zugrichtung der Bandage parallel zueinander angeordneten und wenigstens einseitig durch textile Flächen gebilde abgedeckte, gedehnte Elastomerfäden, wobei die Elastomerfäden mit dem bzw. den textilen Flächengebilde (n) bzw. die textilen Flächengebilde miteinander verbunden bzw. verklebt sind und die Bandage infolge Relaxation der eingebundenen Elastomerfäden gestaucht ist, dadurch gekennzeichnet, daß das bzw. die textile(n) Flächengebilde ein insbesondere leichtgewichtiger Vliesstoff ist bzw. sind und die Elastomerfäden ein- oder mehrfach umsponnen sind und einen Dehnungsbetrag von 30 bis 400 % aufweisen, und der bzw. die Vliesstoff(e) durch stellenweise thermische Verklebung der Fasern verdichtet und derart mit den umsponnenen Elastomerfäden und gegebenenfalls mit miteinander verbunden sind, daß die Elastomerfäden fixiert, jedoch in den nicht verdichteten Bereichen beweglich angeordnet sind.

2. Elastische Bandage nach Anspruch 1, dadurch gekennzeichnet, daß der Vliesstoff einen Anteil von wenigstens 10 Gew.%, bezogen auf das gesamte Fasergewicht, an thermoplastischen Bindefasern enthält und daß die umsponnenen Elastomerfäden parallel zueinander in einem Abstand von 1 bis 10 mm angeordnet sind.

3. Elastische Bandage nach einem der Ansprüche 1 oder 2, dadurch gekennzeichnet, daß die Vliesstoffe aus Florlagen mit unterschiedlichen Faserzusammensetzungen aufgebaut sind, wobei wenigstens zwei Schichten vorgesehen sind und die Außenschicht aus 100 % thermoplastischen Bindefasern besteht und die innere, den Elastomerfäden zugewandte Schicht, aus wenigstens 10 Gew.% hydrofilen Fasern, wobei der Anteil an thermoplastischen Bindefasern von außen nach innen progressiv abnimmt.

4. Elastische Bandage nach einem der Ansprüche 1 bis 3, dadurch gekennzeichnet, daß der Vliesstoff ein Wirrfaservliesstoff ist.

5. Elastische Bandage nach einem der Ansprüche 1 bis 3, dadurch gekennzeichnet, daß der Vliesstoff aus quergelegten Florlagen besteht und wenigstens eine Abdeckschicht aus Längsflor vorgesehen ist.

6. Elastische Bandage nach Anspruch 5, dadurch gekennzeichnet, daß die Außenfläche des Vliesstoffes eine Längsflorlage enthält.

7. Elastische Bandage nach einem der Ansprüche 1 bis 3, dadurch gekennzeichnet, daß ein aus mehreren Lagen zusammengesetzter Vliesstoff vorgesehen ist, der wenigstens eine Lage Spinnvliesstoff, Naßvliesstoff und/oder Trockenvliesstoff enthält.

8. Elastische Bandage nach einem der Ansprüche 1 bis 7, dadurch gekennzeichnet, daß 4 bis 50 % der Oberfläche des Vliesstoffes durch thermische Verschweißung verdichtet ist.

9. Elastische Bandage nach Anspruch 8, dadurch gekennzeichnet, daß 5 bis 25 % der Oberfläche des Vliesstoffes durch thermische Verschweißung verklebt ist.

10. Elastische Bandage nach einem der Ansprüche 1 bis 9, dadurch gekennzeichnet, daß sie durch Punktschweißung verklebt ist.

11. Verfahren zur Herstellung von elastischen Bandagen nach einem der Ansprüche 1 bis 10, dadurch gekennzeich net, daß umsponnene Elastomerfäden (4) unter Zugspannung abgezogen und in einem Abstand zueinander parallel angeordnet und beidseitig mit mindestens einer Vliesstoff- oder Florbahn (6) abgedeckt werden, die Schichten dann durch einen beheizten Kalander geführt werden, der eine gravierte, beheizte Kalanderwalze (7) und eine glatte, ebenfalls beheizte Stahlwalze (8) aufweist, wobei die Vliesstoff- bzw. Florschichten miteinander und mit den umsponnenen Elastomerfäden durch stellenweise thermische Verschweißung verklebt werden, wobei die Elastomerfäden fest, jedoch beweglich eingebunden werden und daß das vereinigte Bahnenmaterial dann durch eine wässrige, gegebenenfalls Zusätze wie Antischaummittel, Tenside, Mikrobizide, bakteriostatisch- bzw. fungistatisch wirkende Stoffe, Weichmacher, Kunststoff-Dispersionen oder dgl. enthaltende Flotte (9), die eine Temperatur von 60 bis 100°C aufweist, geführt, zwischen Quetschwalzen (11 und 12) abgepreßt und dann in einen Strahlungsoder Kontakttrockner (23) getrocknet wird, wobei eine Stauchung des Materials eintritt.

12. Verfahren nach Anspruch 11, dadurch gekennzeichnet, daß umsponnene Elastomerfäden mit einer Dehnung von 3. bis 400 % verwendet werden, die in einem Abstand von 1 bis 15 mm zwischen den beiden Vliesstoff- bzw. Florlagen (6) parallel zueinander angeordnet werden.

13. Verfahren nach einem der Ansprüche 11 oder 12, dadurch gekennzeichnet, daß 4 bis 50 % der Oberfläche der Abdeckvliessteffe thermisch durch Punktschweißung verfestigt sind.

**Claims**

An elastic bandage for fixing parts of the body, comprising stretched elastomeric filaments which are arranged parallel to one another in the direction of tension of the bandage and are covered on at least one side by textile sheetlike structures, the elastomeric filaments being bonded adhesively or otherwise to the textile sheetlike structure(s), the textile sheetlike structures being bonded adhesively or otherwise to one another when there are more than one, and the bandage being buckled as a consequence of the relaxation of the incorporated elastomeric filaments, characterized in that the or each textile sheetlike structure is a particularly lightweight bonded fibre web and the elastomeric filaments have been wrapped one or more times and have a degree of stretch of 30 to 400%, and that the bonded fibre web(s) have been compacted by local thermal bonding of the fibres and been bonded to the wrapped elastomeric filaments and where appropriate to one another in such a way that the elastomeric filaments have been arranged to be fixed in the compacted areas, but to be mobile in the non-compacted areas.

2. An elastic bandage according to claim 1, characterized in that the bonded fibre web contains less than 10% by weight, based on the total fibre weight, of the thermoplastic binder fibres and that the wrapped elastomeric filaments are arranged parallel to one another a distance of 1 to 10 mm apart.

3. An elastic bandage according to claim 1 or 2, characterized in that the bonded fibre webs are composed of layers of card web having different fibre compositions, at least two layers being provided and the outer layer consisting to the extent of 100% of thermoplastic binder fibres and the inner layer, which faces the elastomeric filaments, consisting to the extent of at least 10% by weight of hydrophilic fibres, the proportion of thermoplastic binder fibres progressively decreasing from out to in.

4. An elastic bandage according to any of claims 1 to 3, characterized in that the bonded fibre web is a random web.

5. An elastic bandage according to any of claims 1 to 3, characterized in that the bonded fibre web consists of cross-laid layers of card web and at least one top layer of card web laid in the longitudinal direction is provided.

6. An elastic bandage according to claim 5, characterized in that the outer surface of the bonded fibre web contains a layer of card web laid in the longitudinal direction.

7. An elastic bandage according to any of claims 1 to 3, characterized in that there is provided a bonded fibre web which is composed of a plurality of layers and which contains at least one layer of spunbonded material, wet-laid material and/or dry-laid material.

8. An elastic bandage according to any one of claims 1 to 7, characterized in that 4 to 50% of the surface of the bonded fibre web is compacted by thermal welding.

9. An elastic bandage according to claim 8, characterized in that 5 to 25% of the surface of the bonded fibre web is adhesively bonded by thermal welding.

10. An elastic bandage according to any of claims 1 to 9, characterized in that it is adhesively bonded by spot welding.

11. A method of manufacture of an elastic bandage according to any of claims 1 to 10, characterized in that wrapped elastomeric filaments (4) are drawn off under tension and are arranged parallel to one another a distance apart and are covered on both sides with at least one bonded fibre or card web (6), the layers are then passed through a hot calender which comprises an engraved, hot calender roll (7) and a smooth, likewise hot steel roll (8), the bonded fibre or card web layers being adhesively bonded to one another and to the wrapped elastomeric filaments by local thermal welding, the elastomeric filaments being bonded in to be fixed but mobile, and that the combined web material is then passed through an aqueous liquor (9) which may contain one or more additives, for example antifoams, surfactants, microbicides, bacteriostatic or fungistatic active substances, softeners or plastics dispersions, and has a temperature of 60 to 100°C, is squeezed off between squeeze rollers (11 and 12) and is then dried in a radiation or contact dryer (23), in the course of which buckling of the material occurs.

12. A method according to claim 11, characterized in that the wrapped elastomeric filaments used have an elongation of 30 to 400% and are arranged parallel to one another a distance of 1 to 15 mm apart between the two bonded fibre or card web layers (6).

13. A method according to claim 11 or 12, characterized in that 4 to 50% of the surface of the topmost bonded fibre webs are consolidated thermally by spot welding.


## Revendications

1. Bandage élastique pour fixer des parties du corps, constitué de filaments élastomères allongés disposés parallèlement les uns aux autres et recouverts, au moins d'un côté, par des éléments textiles, les filaments élastomères étant reliés aux éléments textiles et les éléments textiles étant reliés ensemble par collage, et le bandage étant comprimé par suite de la relaxation des filaments élastomères incorporés, caractérisé en ce que le ou les élément(s) textile(s) sont constitués d'une nappe de fibres particulièrement légères et que les filaments élastomères subissent un guipage simple ou multiple et présentent un taux d'allongement de 30 à 400% et en ce que la ou les nappe(s) de fibres sont compactées par collage thermique par points des fibres et sont fixées aux filaments élastomères guipés et, éventuellement entre elles, de manière à ce que les filaments élastomères soient fixés tout en restant mobiles dans les zones non compactées.

2. Bandage élastique selon la revendication 1, caractérisé en ce que la nappe de fibres contient une proportion d'au moins 10% en poids, par rapport au poids total de fibres, de fibres de liaison thermoplastiques, et en ce que les filaments d'élastomère guipés sont disposés parallèlement les uns aux autres et à des intervalles de 1 à 10 mm.

3. Bandage élastique selon l'une des revendications 1 ou 2, caractérisé en ce que les nappes de fibres sont constituées de voiles de fibres ayant des compositions de fibres différentes, au moins deux couches étant prévues et la couche extérieure étant constituée de 100% de fibres de liaison thermoplastiques et la couche inférieure tournée vers les filaments élastomères étant constituée d'au moins 10% en poids de fibres hydrophiles, la proportion de fibres de liaison thermoplastiques diminuant progressivement de l'intérieur vers l'extérieur.

4. Bandage élastique selon l'une des revendications 1 à 3, caractérisé en ce que la nappe de fibres est une nappe avec des fibres disposées au hasard.

5. Bandage élastique selon l'une des revendications 1 à 3, caractérisé en ce que la nappe de fibres est constituée de voiles de fibres disposées transversalement et qu'il est prévu au moins une couche de recouvrement constituée d'un voile longitudinal.

6. Bandage élastique selon la revendication 5, caractérisé en ce que la surface extérieure de la nappe de fibres comporte une couche de voile longitudinale.

7. Bandage élastique selon l'une des revendications 1 à 3, caractérisé en ce qu'il est prévu une nappe de fibres constituée d'une ou plusieurs couches, qui contient au moins une couche de nappes de fibres filées, de nappes de fibres préparées par voie humide et/ou par voie sèche.

8. Bandage élastique selon l'une des revendications 1 à 7, caractérisée en ce que 4 à 50% de la surface de la nappe de fibres sont compactés par soudage thermique.

9. Bandage élastique selon la revendication 8, caractérisé en ce que 5 à 25% de la surface de la nappe de fibres sont assemblés par soudage à chaud.

10. Bandage élastique selon l'une des revendications 1 à 9, caractérisé en ce que l'assemblage s'effectue par soudage par points.

11. Procédé de fabrication de bandages élastiques selon l'une des revendications 1 à 10, caractérisé en ce que les filaments élastomères guipés (4) sont étirés sous tension de traction et sont disposés parallèlement les uns aux autres à un certain intervalle et sont recouverts, sur les deux faces, d'au moins une bande de nappe ou de voile de fibres (6), ces couches étant amenées ensuite à une calandre chauffée comportant un cylindre de calandrage chauffé avec tracé d'impression (7) et un cylindre d'acier lisse, également chauffé (8), les couches de nappes ou de voiles de fibres étant assemblées ensemble et aux filaments élastomères guipés par soudage à chaud par points, les filaments élastomères se trouvant ainsi solidement fixés, tout en conservant leur mobilité, et en ce que le matériau en bandes ainsi assemblé est amené dans un bain aqueux comportant, éventuellement, des additifs tels que produits anti-moussants, tensio-actifs, microbicides, substances à effet bactériostatique ou fongistatique, assouplissants, dispersion de matières plastiques ou analogues (9) ayant une température de 60° à 100°C, puis sont pressés entre des cylindres d'essorage (11) et (12) et sont séchés ensuite dans un sécheur par rayonnement ou à contact (23), ceci provoquant une compression du matériau.

12. Procédé selon la revendication 11, caractérisé en ce que les filaments élastomères guipés sont utilisés avec un allongement de 30 à 400% et sont disposés parallèlement les uns aux autres et entre les deux couches de voile ou de nappe de fibres (6) avec des intervalles de 1 à 15 mm.

13. Procédé selon l'une des revendications 11 ou 12, caractérisé en ce que 4 à 50% de la surface des nappes de fibres de recouvrement sont renforcés par soudage à chaud par points.

Fig. 1

Fig. 2a

Fig. 2b

Fig. 3